# EUROPEAN PATENT SPECIFICATION

(11) **EP 2 707 088 B1**
(45) Date of publication and mention of the grant of the patent: **07.07.2021**
(21) Application number: 12729208.4
(22) Date of filing: 10.05.2012
(51) Int. Cl.: A61N 1/32, A61N 1/30

(54) **ELECTROPORATION DEVICE**
ELEKTROPORATIONSVORRICHTUNG
DISPOSITIF D'ÉLECTROPORATION

(30) Priority: 11.05.2011 IT TO20110411
(43) Date of publication of application: 19.03.2014
(73) Proprietor: Fondazione Istituto Italiano di Tecnologia, 16163 Genova (IT); Consiglio Nazionale Delle Ricerche, 00185 Roma (IT)
(72) Inventor: CANCEDDA, Laura, 16128 Genova (IT); RATTO, Gian Michele, 56011 CALCI (Pisa) (IT)
(74) Representative: Deambrogi, Edgardo
(86) International application number: PCT/IB2012/052327
(87) International publication number: WO 2012/153291

(56) References cited:
- WO-A1-03/013615
- US-A- 5 718 246
- US-A- 6 123 701
- US-A1- 2002 072 746
- US-A1- 2004 019 371
- US-A1- 2006 224 192
- US-A1- 2007 156 082
- None

## Description

The present invention relates to an electroporation device. Electroporation is a method used in molecular biology for introducing into cells, by the external application of an electrical field, molecules such as DNA molecules or chemotherapy drugs which would otherwise be unable to pass through the cell membrane. The application of an electrical field to the cell by transmitting electrical pulses serves to create pores in the cell membrane which allow the passage of the material to be introduced into the cell. Electroporation is used, for example, in what are known as transfection processes, in other words processes of introducing exogenous biological material (particularly genetic material, usually including DNA) into eukaryotic cells. Electroporation has recently been used successfully for the in vivo investigation of gene function and neural activity in the brain. In particular, electroporation in utero, in which a set of voltage pulses is used to transfect DNA into neuron precursor cells, has allowed to investigate the prenatal development of the cerebral cortex, of the corpus striatum, and of the hippocampus. Various genes have now been successfully transfected into cells in various areas of the brain such as the telencephalon, the diencephalon, the mesencephalon, the rhombencephalon and the spinal cord.

For the purpose of applying the electrical field required to carry out electroporation, the use of devices comprising a pincer or forceps whose two jaws each carry a respective electrode connected to a corresponding polarity is known. This two-electrode configuration of known electroporation devices has a drawback in that the success rate of electroporation is low in all the points of the cerebral cortex that can easily be analysed for images and in other areas of the brain such as the hippocampus.

US 5 810 762 discloses an electroporation device comprising a support member, a pair of electrodes mounted on the support member in such a way that they can be moved towards or away from each other so as to be positioned on opposite sides of a portion of the body to be subjected to electroporation, a sensor adapted to detect the distance between the electrodes and to supply a corresponding signal, and control means adapted to generate electrical pulses on the two electrodes with an intensity proportional to the distance between the electrodes. This known device has therefore the advantage of increasing the range of action by making it possible to adjust the distance between the electrodes, and of enabling the efficacy of the electroporation to be improved by controlling the electrical pulses applied to the electrodes as a function of the distance between the electrodes.

US 5 718 246 provides an electroporation device comprising a pair of electrodes carried by a forceps-type member. The device disclosed in this document is also provided with a third electrode (more specifically, a "patch clamp" electrode), although this is not used for electroporation, but for making electrical measurements. With such a device, therefore, the direction of the electrical field by means of which the electroporation is carried out is fixed.

The present invention provides an electroporation device according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

It is an object of the present invention to provide an electroporation device which enables the range of action and efficacy of the electroporation to be further improved, and which is inexpensive and easy to handle and use.

This and other objects are fully achieved according to the present invention by means of an electroporation device having the characteristics defined in the characterizing part of the attached independent Claim 1.

Preferred embodiments of the present invention are described in the dependent claims, the content of which is to be considered as an integral and integrating part of the following description.

Briefly, the invention is based on the idea of providing an electroporation device comprising three electrodes, two of which are carried by a forceps-type member in such a way that they can be moved towards or away from each other, while the third electrode is movable independently of the first two.

The third electrode is preferably carried by a grip member which is separate from the forceps-type member carrying the first two electrodes.

In one embodiment, the two electrodes carried by the forceps are connected to the same polarity, while the third electrode is connected to the other polarity. Alternatively, the two electrodes carried by the forceps can be connected to different polarities, in which case the third electrode is connected to the same polarity as one of the two electrodes carried by the forceps.

Because of the presence of three electrodes, the electroporation device according to the invention evidently allows the range of action of the device to be extended, as the spatial configuration of the electrical field generated by the device can be modified, thus subjecting cells at different points in the brain to electroporation simply by varying the relative position of the third electrode with respect to the first two electrodes and/or by varying the polarities of the three electrodes. The electroporation device according to the invention therefore allows electroporation to be carried out at different points in the brain, such as the cerebral cortex, the hippocampus and the cerebellum, in a more efficient and reliable way. In particular, in the case of the cerebral cortex, the electroporation device according to the invention can be used to transfect cells located at points which can easily be investigated in vivo by means of imaging techniques.

The electroporation device according to the invention is also economical to produce and can advantageously be made by using a forceps-type electroporation device already available on the market and adding a third electrode.

Further characteristic and advantages of the present invention will become clear from the following detailed description which is given purely by way of non-limiting example with reference to the attached drawings, in which:
Figure 1 is a perspective view of an electroporation device according to a preferred embodiment of the present invention, made by using a two-electrode electroporation device available on the market;
Figure 2 is a perspective view which shows, on an enlarged scale, the main components of the device of Figure 1; and
Figures 3A, 3B and 3C are schematic representations of three different possible arrangements of the electrodes of the electroporation device according to the invention, for the electroporation of the cells of the cerebral cortex, the hippocampus and the cerebellum, respectively, of an embryo, each arrangement of the electrodes being shown in both the sagittal plane and the coronal plane.

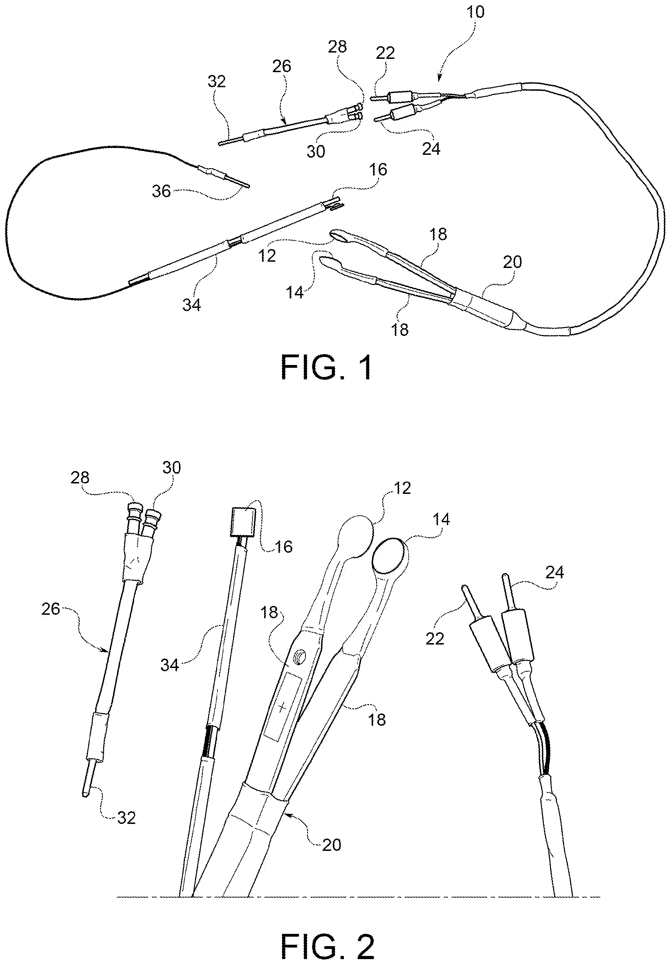

With initial reference to Figures 1 and 2, an electroporation device according to an embodiment of the present invention is generally indicated 10. The electroporation device 10 (referred to hereinafter simply as the "device 10") comprises a first electrode 12, a second electrode 14 and a third electrode 16. The first two electrodes 12 and 14 are, for example, mounted at the ends of the jaws 18 of a forceps 20 of a known type, in such a way that they can be moved towards or away from each other. The first and second electrodes 12 and 14 can be connected to different polarities or can both be connected to the same polarity. In the illustrated embodiment, the forceps 20 which is used can be a forceps available on the market, in which one of the electrodes 12 and 14 can be connected to one polarity and the other electrode can be connected to the other polarity, by means of respective connectors 22 and 24. Therefore, if both electrodes 12 and 14 are to be connected to the same polarity (as in the example shown in Figures 1 and 2), the device 10 will further comprise an adaptor 26 having a pair of input connectors 28 and 30 intended to be connected to the connectors 22 and 24 of the forceps 20, and a single output connector 32 intended to be connected to one of the two polarities. In the illustrated embodiment, the third electrode 16 is mounted at the end of a grip member 34, which is made for example in the shape of a rod, and is made for example of plastic material, and is connectable to the respective polarity by means of a connector 36. The grip member 34 is a separate member from the forceps 20, or is at least a member which is movable with respect to the forceps 20, in such a way that the position of the third electrode 16 can be varied with respect to the first two electrodes 12 and 14 in order to vary the spatial configuration of the electrical field generated by the device. The three-electrode configuration of the device 10 allows to vary not only the relative position of the third electrode 16 with respect to the first two electrodes 12 and 14, but also the polarities of the electrodes 12, 14 and 16. The device 10 further comprises electrical pulses generation means adapted to generate electrical pulses of suitable duration and intensity, to be transmitted to the electrodes 12, 14 and 16 through the connectors 32 and 36. These electrical pulses generation means are not shown in the drawings and will not be described in detail herein, since they are of a known type and are therefore not part of the present invention.

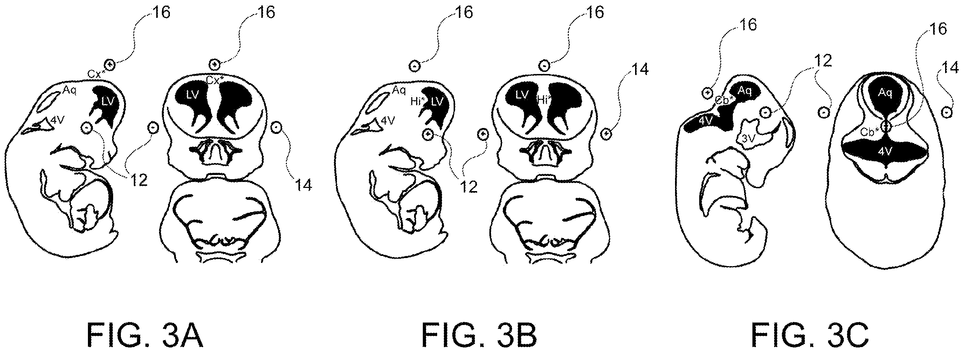

Finally, Figures 3A, 3B and 3C show some possible arrangements of the three electrodes 12, 14 and 16 (shown purely schematically in these drawings) which can be provided by the device 10, in the configuration in which the first and the second electrodes 12 and 14 are both connected to the same polarity, for the electroporation of cerebral cells of an embryo. Each arrangement is shown in both the sagittal plane and the coronal plane. Figure 3A shows, in particular, an arrangement of the electrodes which can be used for the electroporation of the cells of the cerebral cortex of the embryo, in which the first two electrodes 12 and 14 are connected to the negative polarity, while the third electrode 16 is connected to the positive polarity. Figure 3B shows, in particular, an arrangement of the electrodes which can be used for the electroporation of the cells of the hippocampus of the embryo, in which the first two electrodes 12 and 14 are connected to the positive polarity, while the third electrode 16 is connected to the negative polarity. Figure 3C shows, in particular, an arrangement of the electrodes which can be used for the electroporation of the cells of the cerebellum of the embryo, in which the first two electrodes 12 and 14 are connected to the positive polarity, while the third electrode 16 is connected to the negative polarity.

Naturally, the principle of the invention remaining unchanged, the embodiments and the details of construction may vary widely from what has been described and illustrated purely by way of non-limiting example. However, it is to be understood that the present invention is solely defined by the appended claims.

## Claims

1. Electroporation device (10) comprising a forceps-type member (20) and first and second electrodes (12, 14) carried by the forceps-type member (20) so that they can be moved towards and away from each other,
**characterized in that** it further comprises a third electrode (16) configured to be movable independently of the first and second electrodes (12, 14) to allow to modify the spatial configuration of an electrical field generated by the device (10).

2. Electroporation device according to Claim 1, wherein the first and second electrodes (12, 14) are designed to be both connected to a same polarity, whereas the third electrode (16) is designed to be connected to the other polarity.

3. Electroporation device according to Claim 1 or Claim 2, wherein the first and second electrodes (12, 14) are designed to be connected to different polarities.

4. Electroporation device according to any of the preceding claims, further comprising a grip member (34) which is separate from the forceps-type member (20) and carries the third electrode (16).

## Patentansprüche

1. Elektroporationsvorrichtung (10), umfassend ein Pinzettenelement (20) sowie eine erste und eine zweite Elektrode (12, 14), die derart von dem Pinzettenelement (20) getragen werden, dass sie aufeinander zu und voneinander weg bewegt werden können,
**dadurch gekennzeichnet, dass** sie ferner eine dritte Elektrode (16) umfasst, die so konfiguriert ist, dass sie unabhängig von der ersten und der zweiten Elektrode (12, 14) beweglich ist, um die räumliche Konfiguration eines von der Vorrichtung (10) erzeugten elektrischen Feldes modifizieren zu können.

2. Elektroporationsvorrichtung nach Anspruch 1, wobei die erste und die zweite Elektrode (12, 14) so ausgelegt sind, dass sie beide mit derselben Polarität verbunden werden, während die dritte Elektrode (16) so ausgelegt ist, dass sie mit der anderen Polarität verbunden wird.

3. Elektroporationsvorrichtung nach Anspruch 1 oder Anspruch 2, wobei die erste und die zweite Elektrode (12, 14) so ausgelegt sind, dass sie mit unterschiedlichen Polaritäten verbunden werden.

4. Elektroporationsvorrichtung nach einem der vorhergehenden Ansprüche, ferner umfassend ein Griffelement (34), das von dem Pinzettenelement (20) getrennt ist und die dritte Elektrode (16) trägt.

## Revendications

1. Dispositif d'électroporation (10) comprenant un élément de type forceps (20) et des première et deuxième électrodes (12, 14) portées par l'élément de type forceps (20), de sorte qu'elles peuvent être rapprochées et éloignées l'une de l'autre,
**caractérisé en ce qu'**il comprend en outre une troisième électrode (16) configurée pour pouvoir être déplacée indépendamment des première et deuxième électrodes (12, 14) afin de permettre de modifier la configuration spatiale d'un champ électrique généré par le dispositif (10).

2. Dispositif d'électroporation selon la revendication 1, dans lequel les première et deuxième électrodes (12, 14) sont conçues pour être toutes deux connectées à une même polarité, tandis que la troisième électrode (16) est conçue pour être connectée à l'autre polarité.

3. Dispositif d'électroporation selon la revendication 1 ou la revendication 2, dans lequel les première et deuxième électrodes (12, 14) sont conçues pour être toutes deux connectées à des polarités différentes.

4. Dispositif d'électroporation selon l'une quelconque des revendications précédentes, comprenant en outre un élément de préhension (34) qui est séparé de l'élément de type forceps (20) et porte la troisième électrode (16).
